# EUROPÄISCHE PATENTSCHRIFT

(11) **EP 0 529 341 B1**
(45) Veröffentlichungstag und Bekanntmachung des Hinweises auf die Patenterteilung: **12.07.1995**
(21) Anmeldenummer: 92113076.1
(22) Anmeldetag: 31.07.1992
(51) Int. Cl.: A61B 17/34, A61M 25/06

(54) **Anästhesiebesteck**
Set of instruments for anaesthesia
Trousse pour l'anestésie

(30) Priorität: 28.08.1991 DE 4128530
(43) Veröffentlichungstag der Anmeldung: 03.03.1993
(73) Patentinhaber: VYGON GMBH & CO KG, D-52070 Aachen (DE)
(72) Erfinder: Heiliger, Raymund, Dr., W-5120 Herzogenrath (DE); Gross, Joachim, Dr., W-7032 Sindelfingen (DE)
(74) Vertreter: Bauer, Hubert, Dipl.-Ing.

(56) Entgegenhaltungen:
- EP-A- 0 359 987
- WO-A-89/00436
- WO-A-90/14124
- DE-C- 3 922 406

## Beschreibung

Die Erfindung betrifft ein Anästhestiebesteck aus einer Epiduralkanüle, einem Epiduralkatheter und einer Spinalkanüle. Die einen Mandrin enthaltende Epiduralkanüle ist an ihrem distalen Ende mit einer radialen Austrittsöffnung für den Epiduralkatheter und mit einer axialen Austrittsöffnung für die Spinalkanüle versehen.

Ein derartiges aus der DE 39 22 406 C1 bekanntes Anästhesiebesteck besteht aus einer im wesentlichen geraden Epiduralkanüle mit angeschliffener Austrittsöffnung, einer Spinalkanüle, die länger und dünner als die Epiduralkanüle ausgebildet ist und in diese so einschiebbar ist, daß sie mit ihrem distalen Ende aus der Epiduralkanüle vorsteht, und einem durch die Epiduralkanüle schiebbaren Epiduralkatheter. Dabei ist die Spitze der Epiduralkanüle abgebogen und die Durchtrittsöffnung für den Epiduralkatheter radial seitwärts gerichtet. In der äußeren Wand der Krümmung der Spitze ist eine auf die Längsmittelachse der Epiduralkanüle zentrierte Austrittsöffnung für die Spinalkanüle vorgesehen. Diese Austrittsöffnung ist kleiner als der Außendurchmesser des Epiduralkatheters.

Die Handhabung des bekannten Anästhesiebestecks ist für den Anwender nur dann einfach, wenn die Spitze der Spinalkanüle zwangsläufig in den Spinalkanal geradlinig eintritt und der Epiduralkatheter zwangsläufig in den Epiduralraum eingeführt werden kann. Während die entsprechende Einführung des Epiduralkatheters grundsätzlich keine Schwierigkeiten bereitet, erfordert die wesentlich dünner als die Epiduralkanüle ausgebildete Spinalkanüle zur ordnungsgemäßen Einbringung ihrer Spitze in den Spinalkanal besondere Vorkehrungen, damit insbesondere die Spitze der Spinalkanüle die axiale Austrittsöffnung in der Epiduralkanüle nicht verfehlt. Für das bekannte Anästhesiebesteck wird daher vorgeschlagen, auf der Spinalkanüle mit Abstand zu ihrer Spitze einen koaxialen Führungsring zu befestigen, dessen Außendurchmesser geringfügig kleiner als der Innendurchmesser der Epiduralkanüle ist. Weil ein solcher Führungsring offensichtlich eine Schräglage der Spinalkanüle innerhalb der Epiduralkanüle nicht auszuschließen vermag, wird zur Verbesserung der Führung empfohlen, den Führungsring als umfangmäßig geschlossenes kreiszylindrisches Rohr auszubilden, das vorzugsweise aus einem Kunststoffschlauch besteht, der sich unter Freilassung eines spitzennahen Kanülenabschnitts über die gesamte Länge der Spinalkanüle erstrecken soll. Die sichere Führung und Plazierung der Spinalkanüle erfordert somit einen erheblichen zusätzlichen Aufwand, durch den die Herstellung des bekannten Anästhesiebesteckes verteuert wird.

Der Erfindung liegt die Aufgabe zugrunde, ein Anästhesiebesteck der eingangs beschriebenen Art so auszubilden, daß es einfacher handhabbar ist und sich damit die Spinalkanüle ebenso sicher führen und plazieren läßt wie der Epiduralkatheter, ohne daß dazu die Spinalkanüle mit einem Hilfsmittel versehen oder in anderer Weise dem größeren Innendurchmesser der Epiduralkanüle angepaßt werden muß.

Zur Lösung dieser Aufgabe wird von einem Anästhesiebesteck der im Oberbegriff des Anspruchs 1 genannten gattungsgemäßen Art ausgegangen, welches erfindungsgemäß die in seinem kennzeichenden Teil angegebenen Merkmale aufweist.

Durch die erfindungsgemäße Aussparung im Mandrin enthält die Spinalkanüle eine exakte Führung, ohne dazu eines zusätzlichen Hilfsmittels zu bedürfen. Dadurch ist aber auch die Handhabung des Anästhesiebesteckes erleichtert, weil der das Lumen der Epiduralkanüle zur Vermeidung von Ausstanzungen im übrigen ausfüllende Mandrin während der Einbringung der Spinalkanüle und der Durchführung der Spinalanästhesie in der Epiduralkanüle verbleiben kann und erst unmittelbar vor der Einbringung des Epiduralkatheters aus der Epiduralkanüle zu entfernen ist.

Nach einer Ausgestaltung der Erfindung ist die Aussparung durch eine zur Längsachse des Mandrins koaxiale Bohrung erzeugt.

Ist die axiale Austrittsöffnung für die Spinalkanüle auf die Längsmittelachse der Epiduralkanüle zentriert, fluchtet die zur Längsachse des Mandrins koaxiale Bohrung zwangsweise mit der axialen Austrittsöffnung, so daß bei gleichem Durchmesser der Aussparung und der Projektion der Austrittsöffnung die Laibung der Aussparung stufenlos in die Laibung der axialen Durchtrittsöffnung übergeht.

Um die Spitze der Spinalkanüle gezielt der axialen Austrittsöffnung in der Epiduralkanüle zuzuleiten, kann es nach einer anderen Ausgestaltung der Erfindung ausreichen, die Aussparung im Mandrin durch eine zu seiner Längsachse parallele Nut zu erzeugen.

Der seitliche Öffnungsquerschnitt der Nut im Mandrin wird durch die Wandung der Epiduralkanüle abgesperrt, so daß ebenso wie durch eine axiale Bohrung im Mandrin ein Führungskanal für die Spinalkanüle mit Hilfe der Nut entsteht, durch welche die Spitze der Spinalkanüle zwangsläufig der axialen Austrittsöffnung in der Epiduralkanüle zugeleitet wird.

Schließlich sieht eine Ausgestaltung der Erfindung noch vor, daß die Aussparung durch eine Querschnittsreduzierung des Mandrins über eine Teillänge zwischen der axialen Austrittsöffnung der Epiduralkanüle in Richtung zum proximalen Ende erweitert ist.

Durch die erweiterte Aussparung verbleibt innerhalb der Epiduralkanüle auch in Anwesenheit des Mandrins und der Spinalkanüle ein freier Hohlraum, der die Ermittlung eines Druckverlustes während der Spinalanästhesie erleichtert, da dieser Hohlraum dem Analgetikum einen wesentlich geringeren Widerstand entgegensetzt als der von der Dura ummantelte Inhalt im Spinalkanal.

In der Zeichnung ist als Ausführungsbeispiel des erfindungsgemäßen Anästhesiebesteckes jeweils eine teilweise geschnittene Seitenansicht eines distalen Endstückes dargestellt. Es zeigen:
- Fig. 1: eine Epiduralkanüle,
- Fig. 2: einen Mandrin,
- Fig. 3: die Epiduralkanüle mit dem Mandrin und einer Spinalkanüle im eingeschobenen Zustand.

Eine in an sich bekannter Weise aus einem geraden Rohrstück gebildete Epiduralkanüle 1 ist an ihrem distalen Ende abgebogen. Durch einen zu ihrer Längsmittelachse parallelen Schliff ist die Epiduralkanüle 1 mit einer radialen Austrittsöffnung 2 versehen. Im Krümmungsbereich ist ein konvexes Wandteil 3 der Epiduralkanüle 1 mit einer axialen Austrittsöffnung 4 versehen, die auf die strichpunktiert dargestellte Längsmittelachse 5 der Epiduralkanüle 1 zentriert ist.

Da mit der Spitze der Epiduralkanüle 1 die Haut und das Muskelgewebe durchstochen werden müssen, wird zur Verhinderung einer Ausstanzung von Haut- und Gewebeteilen in die Epiduralkanüle 1 ein Mandrin 6 eingeschoben, der mit einem distalen Endstück 7 einen bündigen Verschluß für die radiale Austrittsöffnung 2 der Epiduralkanüle 1 bildet.

Der Mandrin 6 ist mit einer Aussparung 8 in Form einer zu seiner Längsachse koaxialen Bohrung versehen, deren Querschnitt der Projektionsfläche der axialen Austrittsöffnung 4 in der Epiduralkanüle entspricht und die ebenso wie diese auf die Längsmittelachse der Epiduralkanüle 1 zentriert ist. Die Aussparung 8 ist über eine Teillänge des Mandrins 6 zwischen der axialen Austrittsöffnung 4 in Richtung zum proximalen Ende dadurch erweitert, daß der Mandrin 6 über die Hälfte seines Querschnittes abgeflacht ist.

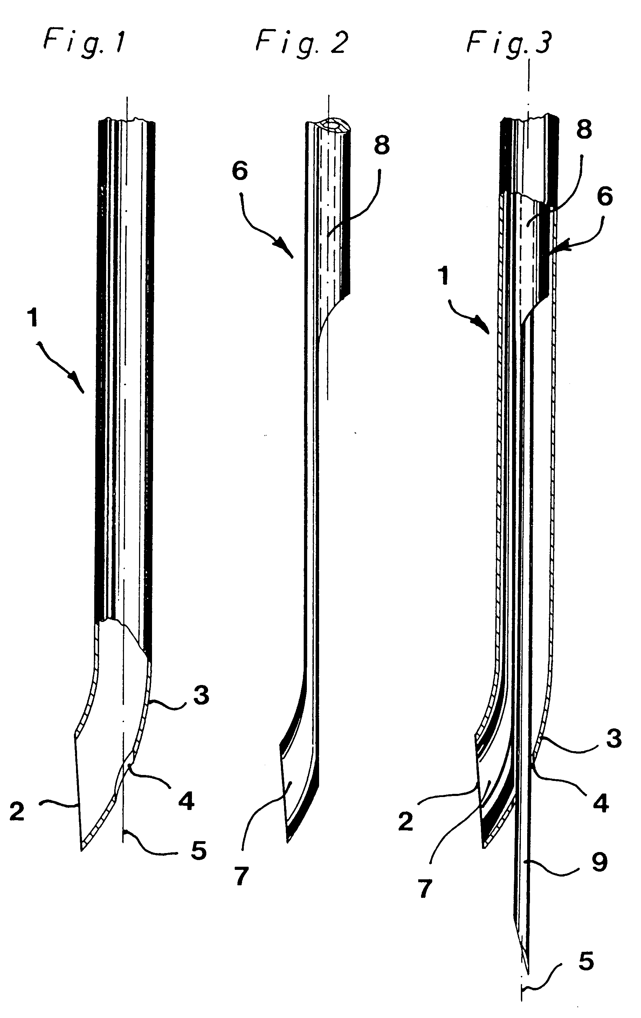

Wie Fig. 3 veranschaulicht, dient der in der Epiduralkanüle 1 plazierte Mandrin 6 nicht nur zum Verschluß der radialen Austrittsöffnung 2, sondern darüber hinaus zur Führung einer Spinalkanüle 9 durch die Epiduralkanüle 1 und durch deren axiale Austrittsöffnung 4. Die der Spinalkanüle 9 angepaßte Aussparung 8 in Form der axialen Bohrung im Mandrin 6, deren Durchmesser nur geringfügig größer ist als der Außendurchmesser der Spinalkanüle 9, gewährleistet eine sichere Führung der Spinalkanüle 9 durch die axiale Durchtrittsöffnung 4 in der Epiduralkanüle 1.

## Patentansprüche

1. Anästhestiebesteck aus einer Epiduralkanüle (1), einem Epiduralkatheter und einer Spinalkanüle (9), wobei die einen Mandrin (6) enthaltende Epiduralkanüle an ihrem distalen Ende mit einer radialen Austrittsöffnung (2) für den Epiduralkatheter und mit einer axialen Austrittsöffnung (4) für die Spinalkanüle versehen ist, dadurch gekennzeichnet, daß der Mandrin (6) über seine Länge mit einer zur axialen Austrittsöffnung (4) der Epiduralkanüle (1) koaxialen Aussparung (8) versehen ist, deren Querschnitt dem Außendurchmesser der Spinalkanüle (9) angepaßt ist.

2. Anästhesiebesteck nach Anspruch 1, dadurch gekennzeichnet, daß die Aussparung (8) durch eine zur Längsachse des Mandrins (6) koaxiale Bohrung erzeugt ist.

3. Anästhesiebesteck nach Anspruch 1, dadurch gekennzeichnet, daß die Aussparung (8) durch eine zur Längsachse des Mandrins (6) parallele Nut erzeugt ist.

4. Anästhesiebesteck nach einem der Ansprüche 1 bis 3, dadurch gekennzeichnet, daß die Aussparung (8) durch eine Querschnittsreduzierung des Mandrins (6) über eine Teillänge zwischen der axialen Austrittsöffnung (4) der Epiduralkanüle (1) in Richtung zum proximalen Ende erweitert ist.

## Claims

1. A set of anaesthesia instruments comprising an epidural cannula (1), an epidural catheter and a spinal cannula (9), in which respect the epidural cannula, which contains a mandrin (6), is at its distal end provided with a radial outlet opening (2) for the epidural catheter and with an axial outlet opening (4) for the spinal cannula**, characterized in that** the mandrin (6) is provided over its length with a cut-out (8), which is coaxial to an axial outlet opening (4) of the epidural cannula (1) and the cross-section of which is matched to the outside diameter of the spinal cannula (9).

2. A set of anaesthesia instruments according to claim 1, **characterized in that** the cut-out (8) is produced by way of a bore which is coaxial to the longitudinal axis of the mandrin (6).

3. A set of anaesthesia instruments according to claim 1, **characterized in that** the cut-out (8) is produced by way of a groove which is parallel with the longitudinal axis of the mandrin (6).

4. A set of anaesthesia instruments according to one of claims 1 to 3, **characterized in that** the cut-out (8) is widened in the direction of the proximal end by a cross-sectional reduction of the mandrin (6) over a partial length between the axial outlet opening (4) of the epidural cannula (1).

## Revendications

1. Trousse pour anesthésie, composée d'une canule épidurale (1), d'un cathéter épidural et d'une canule spinale (9), et dans laquelle la canule épidurale, qui contient un mandrin (6), est pourvue à son extrémité distale d'une ouverture de sortie radiale (2) pour le cathéter épidural et d'une ouverture de sortie axiale (4) pour la canule spinale, cette trousse étant caractérisée par le fait que le mandrin (6) est pourvu, sur sa longueur, d'une partie évidée (8) coaxiale à l'ouverture de sortie axiale (4) de la canule épidurale (1) et dont la section transversale est adaptée au diamètre extérieur de la canule spinale (9).

2. Trousse pour anesthésie selon la revendication 1, caractérisée par le fait que la partie évidée (8) est constituée par un alésage coaxial à l'axe longitudinal du mandrin (6).

3. Trousse pour anesthésie selon la revendication 1, caractérisée par le fait que la partie évidée (8) est constituée par une rainure parallèle à l'axe longitudinal du mandrin (6).

4. Trousse pour anesthésie selon l'une quelconque des revendications 1 à 3, caractérisée par le fait que la partie évidée (8) est agrandie grâce à une réduction de la section transversale du mandrin (6), sur une partie de sa longueur, située entre l'ouverture de sortie axiale (4) de la canule épidurale (1) et l'extrémité proximale, en direction de celle-ci.
